(19) Europäisches Patentamt
European Patent Office
Office européen des brevets

(11) EP 0 458 684 B1

(12) **FASCICULE DE BREVET EUROPEEN**

(45) Date de publication et mention de la délivrance du brevet: **11.11.1998 Bulletin 1998/46**

(21) Numéro de dépôt: **91401278.6**

(22) Date de dépôt: **17.05.1991**

(51) Int. Cl.$^6$: **C07B 37/04**, C07C 313/02 // C07C323/01, C07C323/58, C07C39/24, C07C43/225, C07C49/16, C07C69/63, C07C69/88, C07C211/52, C07C233/15, C07D207/33, C07D231/44, C07D309/36

(54) **Réactif et procédé de perfluoroalkylation de substrats nucléophiles par les perfluoroalcanesulfinates de sodium en milieu oxydant**

Reagenz und Verfahren zur Perfluoralkylierung von nukleophilen Substraten durch Natriumperfluoralkansulfinate in einem oxydierenden Mittel

Reagent and process for the perfluoroalkylation of nucleophilic substrates by sodium perfluoroalkanesulfinates in an oxidising medium

(84) Etats contractants désignés:
**AT BE CH DE DK ES FR GB GR IT LI LU NL SE**

(30) Priorité: **23.05.1990 FR 9006426**

(43) Date de publication de la demande:
**27.11.1991 Bulletin 1991/48**

(73) Titulaire: **RHODIA CHIMIE**
**92408 Courbevoie Cédex (FR)**

(72) Inventeurs:
- **Clavel, Jean-Louis**
  **F-69130 Ecully (FR)**
- **Laurent, Eliane**
  **F-69300 Caluire (FR)**
- **Langlois, Bernard**
  **F-69006 Lyon (FR)**
- **Roidot, Nathalie**
  **F-69006 Lyon (FR)**

(74) Mandataire:
**Ricalens, François et al**
**RHODIA CHIMIE**
**Direction de la Propriéte Industrielle**
**25, quai Paul Doumer**
**92408 Courbevoie Cédex (FR)**

(56) Documents cités:
- **CHEMICAL ABSTRACTS, vol. 112, no. 17, 23 avril 1990, page 662, abrégé no. 157603c, Columbus, Ohio, US; W. HUANG et al.: "Perfluoroalkanesulfinates as perfluoroalkylation reagents", & ACTA CHIM. SIN. (ENGL. ED) 1989, (2), 190-2**
- **CHEMICA ABSTRACTS, vol. 112: Chemical Substance Index, A-Benzoh, janvier-juin 1990, page 76CS, Columbus, Ohio, US; "Acetic acid, compounds. Manganese(2+) salt [638-38-0] alkene perfluoroalkylation by sodium perfluoroalkanesulfinate in presence of, 157603c"**
- **CHEMICAL ABSTRACTS, vol. 112: Chemical Substance Index, Si-Z, janvier-juin 1990, page 10695CS, Columbus, Ohio, US; "Sulfuric acid, compounds. Cerium(4+) salt (2:1) [13590-82-4] alkene perfluoroalkylation by sodium perfluoroalkylation by sodium perfluoroalkanesulfinate in presence of, 157603c"**
- **J. CHEM. SOC. CHEM. COMM., no. 22, 15 novembre 1987, pages 1701-1703, Royal Society of Chemistry; C. WAKSELMAN et al.: "Perfluoroalkylation of anilines in the presence of zinc and sulphur dioxide"**
- **Acta Chimica Sinica English Edition,No 2,1989 pages 190-192**
- **CRC Handbook of Chemistry and Physics,64th Edition,pages D-160-D-162**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.6/3.4

## Description

La présente invention a pour objet un réactif de perfluoroalkylation et un procédé d'application de ce réactif à la perfluoroalkylation des substrats nucléophiles, c'est-à-dire riches en électrons.
Plus particulièrement, la méthode décrite dans le présent brevet concerne la mono ou poly perfluoroalkylation, par un perfluoroalcanesulfinate en milieu oxydant et dans des conditions douces, des composés riches en électrons.

Pour illustrer la richesse de cette catégorie de substrat, on peut citer de façon non limitative :

- les sulfures et disulfures organiques aliphatiques (primaires, secondaires ou tertiaires), aromatiques ou hétérocycliques,
- les thiols,
- les oléfines et les acétyléniques éventuellement activés par des groupements électrodonneurs, comme, par exemple, les énols et leurs dérivés (en particulier éthers et esters d'énols), les énamines et leurs dérivés, les éthers acétyléniques, les ynamines,...,
- les acrylamides,
- les cétones énolisables (β-dicétones, β-cétoesters),
- les cétones ou dérivés d'acides α, β-insaturés, éventuellement cyliques comme, par exemple, l'uracile ou l'uridine,
- les dérivés homo ou hétéroaromatiques, mono ou polycycliques, condensés ou non, substitués de préférence par au moins un groupe électrodonneur par induction et/ou mésomérie comme, par exemple les halogénobenzènes, les phénols, les anilines, les anisoles, le thiophène et dérivés.

Cette technique permet d'accéder, entre autres, à des perfluoroalkyl thioéthers, des β β'-bis-(perfluoroalkyl)-1, 2-diols, des cétones, amides ou acides α-perfluoroalkylés, des dérivés d'énols β-perfluoroalkylés ou des composés aromatiques et hétérocycliques perfluoroalkylés.

Ces composés peuvent être utilisés comme produits biologiquement actifs ou comme leurs précurseurs.

Par exemple, la 3-(trifluorométhyl)aniline entre dans la synthèse des herbicides Fluometron et Norflurazon alors que le 3-(trifluorométhyl) phénol est une des matières premières de l'herbicide Diflufénican. Des herbicides de la famille du Fluothiuron ou les insecticides MB 45950 et MB 46030 (Fiprole) comportent une fonction trifluorométhyl thioéther. La préparation du MB 45950 par cette méthode est d'ailleurs exposée dans le présent brevet.

L'acide (trifluorométhylthio) acétique, accessible par la présente technique, est un intermédiaire dans la synthèse de la céphalosporine antibiotique Cefazaflur (SKF 59962). Le nitrile correspondant permet d'accéder en deux étapes ("one-pot reaction") à la 2-bromo-3-(trifluorométhylthio) pyridine (J. Org. Chem., 1979, 44 (17), 3080). Le 2,3-bis- (trifluorométhyl) acrylamide, produit de trifluorométhylation radicalaire de l'acrylamide, est le précurseur de la 3-(2, 2, 2-trifluoroéthyl) azétidin-2-one, β-lactame utile pour la préparation d'antibiotiques (Tetrahedron Lett., 1989, 30 (1), 109).

L'α, α, α-trifluorothymidine, issu de l'uridine, est un composé antiviral et la 2-(trifluorométhyl) dopamine ou la S-(trifluorométhyl) cystéine, résultant respectivement de la dopamine et de la cystine, sont des inhibiteurs d'enzymes (Bull. Chem. Soc. Jpn, 1986, 59, 447).

L'ω, ω, ω-trifluorométhionine, obtenue à partir de l'homocystine, est, elle, un inhibiteur de l'activation de la méthionine (Can. J. Microbiol., 1986, 12, 143). Les α-(perfluoroalkyl) cétones issues du présent procédé conduisent, par traitement basique, aux β-fluoro-β-perfluoroalkyl-cétones-α, β-insaturées qui sont connues comme d'intéressants diénophiles (Tetrahedron Lett., 1982, 23 (14), 1471). De la même manière, les α-(trifluorométhyl) esters permettent d'obtenir les β-di-fluoroacrylates, utiles dans l'élaboration de polymères présentant d'excellentes propriétés mécaniques et thermiques.

Il existe d'ores et déjà de nombreux réactifs et de nombreuses autres méthodes d'accès aux composés perfluoroalkylés ou aux perfluoroalkyl thioéthers sont décrites soit à partir de précurseurs trichlorométhylés ou de trichlorométhyl thioéthers soit par introduction directe de groupements $C_nF2_n+1$ ($R_f$) ou $R_fS$. Elles présentent toutes des inconvénients qui sont mentionnés lors de la présentation de l'art antérieur qui suit.

Ainsi la fluoration des groupements trichlorométhylés :

a- M. HUDLICKY, "Chemistry of Organic Fluorine Compounds" (2e édition), pp. 96-106, Ellis Horwood Ltd, Chichester (1976).
b- E.A. NODIFF, S. LIPSCHUTZ, P.N. CRAIG, M. GORDON-J. Org. Chem., 1960, 25, 60.
c- L.M. YAGUPOLSKII, M.S. MARENETS-J. Gen. Chem. USSR, 1959, 29 (1), 281.
d- A. SENNING, S.O. LAWESSON-Acta Chim. Scand., 1962, 16, 117 - CA, 57, 7150 h.
e- S.M. SHEIN, T.V. KOSTKINA, Yu.P. MELNIK-USSR Pat. 163.610 (1964).
t- G. BULTEAU- U.S. 3.632.629 (1972) - CA, 76, 112918f.
g- M. MAKOSZA, M. FEDORYNSKI- Synthesis, 1974 (4), 274), qui met en oeuvre la séquence réactionnelle suivante :

$$R\text{-}Y\text{-}CH_3 \xrightarrow[\text{initiateur}]{X.} R\text{-}Y\text{-}CX_3 \xrightarrow[\text{ou } SbF_3 \text{ ou } SbF_3/SbF_5]{\text{HF ou HF}/SbCl_5} R\text{-}Y\text{-}CF_3$$

où

Y = lien valenciel, O, S ;
X = Cl, Br (pour X=Br, cf. If) ;
initiateur = U.V., chaleur, initiateurs chimiques de radicaux ;

Cette technique n'est pas générale car elle suppose que le substrat méthylé de départ supporte la chloration radicalaire et, en particulier, ne comporte pas d'autre substituant $CH_3$. La fluoration par le trifluorure d'antimoine n'est applicable qu'au laboratoire et induit d'importants problèmes de rejet de métaux lourds. La fluoration par HF, facilement industrialisable, nécessite cependant des installations très spécifiques et des mesures de sécurité draconiennes. Lorsque le pentafluorure d'antimoine doit être utilisé comme catalyseur, sa récupération est laborieuse, voire impossible.

On peut noter une voie d'accès direct aux motifs SCCl3 à partir de chloroforme (1g) mais elle implique que le reste de la molécule supporte les milieux très basiques. Du cyanure de sodium, très toxique en est le sous-produit :

$$R\text{-}S\text{-}CN + CHCl_3 + NaOH \xrightarrow{H_2O/TEBA} R\text{-}S\text{-}CCl_3 + NaCN + H_2O$$

L'obtention de groupements $CF_3$ par action du tétrafluorure de soufre sur les acides ou les esters selon la réaction

$$R\text{-}CO_2X + 2\ SF_4 \xrightarrow{HF} R\text{-}CF_3 + SOF_2 + XF \text{ avec X=H, alkyle}$$

nécessite l'emploi de $SF_4$, gazeux (et très toxique) en milieu acide fluorohydrique anhydre (corrosif et toxique) et sous pression. Elle a été, en particulier, appliquée à la synthèse du 3, 3, 3-trifluoropropanoate d'éthyle à partir de monomalonate d'éthyle (Réf. H.M. PETERS, L.O. ROSS, R.L. SIMON, E.H. MARION-J. Chem. Eng. Data, 1971, 16, 376.)

L'obtention de groupements $CF_3$ par action de l'hexafluorure de molybdène sur les acides, leurs chlorures ou leurs esters (réf. M. VAN DER PUY- J. Fluorine Chem., 1979, 13, 375) utilise un agent fluorant peu réactif, toxique et très onéreux, puisqu'issu de la réaction du fluor moléculaire sur MoO2. Les sous-produits sont mal identifiés et posent un sérieux problème de rejet de métaux lourds.

Il convient de faire une mention spéciale des trifluorométhylations à l'aide de bromotrifluorométhane. Le bromotrifluorométhane (Halon F 13B1) est un produit commercial utilisé essentiellement comme extincteur dans les avions et les salles d'ordinateurs. C'est un agent de trifluorométhylation qui présente cependant l'inconvénient d'être gazeux ($Eb_{760}$ = -57° C) et de ne réagir que sous pression supérieure à 3 bars.

L'homolyse thermique de $CF_3Br$ nécessite des températures très élevées du fait de l'énergie importante de la liaison carbone-brome dans ce réactif : les composés aromatiques ne sont trifluorométhylés thermiquement par $CF_3Br$ qu'à 500-600° C et à condition de catalyser la réaction par de l'iode (réf. OLIN Co.-U.S. 4. 038.331 (1975)) (en l'absence d'iode, on observe une bromation aromatique (réf. OLIN Co. - U.S. 3.890.326 (1973).
Cette méthode est évidemment difficile à extrapoler à l'échelle industrielle. Cependant, lorsque le substrat aromatique est très riche en électrons, une activation photochimique permet la trifluorométhylation à température ambiante (réf. SHINGIJUTSU KAIHATSU -Jap. J6 1.027.927 (1984).

Lorsque le nucléophile n'est pas suffisamment réducteur par lui-même, il est nécessaire d'utiliser un réducteur auxiliaire pour engendrer le radical trifluorométhyle. Ce réducteur peut être le zinc, le cuivre ou le radical-anion du bioxyde de soufre (obtenu à partir de $ZnSO_2$, de dithionites ou d'hydroxyméthanesulfinates alcalins). Si le nucléophile est un composé aromatique riche en électrons (phénols, anilines, anisoles, pyrrole,...) (C. WAKSELMAN, M. TORDEUX- J. Chem. Soc., Chem. Commun., 1987, 1701) ou un disulfure (J.L. CLAVEL, B. LANGLOIS, M. TORDEUX, C. WAKSELMAN (RHONE-POULENC), Demande Française 88/16710.), le réducteur auxiliaire ($SO_2^{\bullet -}$ ou Zn ou Cu) peut

être employé en quantités catalytiques. Il convient de mentionner que le rapport isomérique des aromatiques trifluorométhylés n'est pas toujours satisfaisant.

Il faut noter que toutes ces trifluorométhylations en milieu réducteur impliquent que les autres groupements portés par le substrat ne soient pas réductibles dans des conditions opératoires.

Un certain nombre de méthodes ont été développées à partir d'acides perfluoroalcanoïques ou de leurs dérivés qui, par eux-mêmes, se révèlent au moins aussi onéreux que les perfluoroalcanesulfinates de sodium correspondants revendiqués dans la technique faisant l'objet du présent brevet. Il est évident que les dérivés trifluoroacétiques également employés dans la littérature ($CF_3CO_2Na$, $CF_3CO_2Ag$, $(CF_3CO_2)_2$ Hg, $CF_3CO_2R$, $CF_3COCl$, $(CF_3CO)_2O$, $(CF_3CO_2)_2$) sont encore plus coûteux que $CF_3CO2H$.

Ces méthodes utilisent l'oxydation électrochimique des acides perfluoroalcanoïques ou la décomposition de certains sels (Ag,....) ou de peroxydes.

Dans l'édition anglaise d'acta chimica sinica 1989 (2), 190-2, Huang Wei yuan et al ont décrit l'addition de radicaux perfluorés sur des doubles liaisons, en faisant agir les perfluorosulfinates correspondant en présence de tétracétate de plomb mais qu'en revanche les résultats étaient médiocres ou nuls lorsque l'on utilisait des peroxydes (peroxyde de benzoyle ou persulfate de potassium), ainsi qu'on le verra ultérieurement, l'utilisation de catalyseur a permis de vaincre ce prélugé.

C'est pourquoi, un des buts de la présente invention est de fournir un réactif de perfluoroalkylation qui soit moins cher et plus facile à mettre en oeuvre que les précédents.

Un autre but de la présente invention est de fournir un réactif qui permette l'obtention de rapports isomériques différents de ceux obtenus par les réactifs précédents.

Ces buts et d'autres qui apparaitront par la suite sont atteints au moyen d'un réactif perfluoroalkylant constitué par :

a) un ion perfluoroalcanesulfinate, de préférence alcalin et plus préférentiellement de sodium, ou d'un perfluoroalcanesulfinate d'ammonium ou de phosphonium (Le trifluorométhanesulfinate de sodium est plus spécialement préféré) ;

b) un système oxydant choisi parmi les persulfates ou les hydropéroxydes, y compris le réactif de Fenton :

c) un catalyseur choisi parmi les sels métalliques oxydables, les composés du molybdène VI, du vanadium V, de l'osmium VIII (ou du sélénium IV).

Le-système oxydant est monoélectronique, c'est-à-dire susceptible de n'échanger qu'un seul électron avec le système réducteur dans les conditions de l'expérience et tel que le potentiel de la réaction :

$$\text{oxydant} + e^- \text{ ------- réducteur} \qquad (I)$$

soit supérieur ou égal à 1 avantageusement à 1,20 V par rapport à une électrode de référence au calomel saturé (ECS : $Hg/Hg_2Cl_2$). Les oxydants tels que $H_20_2$ seul, les halogènes ou l'hypochlorite de sodium, qui réagissent sur $R_fSO_2Na$ suivant des processus biélectroniques ou des transferts d'atomes conduisant à $R_fSO_3Na$, sont exclus. En revanche, les radicaux hydroxyles engendrés par $H_2O_2 + Fe^{2+}$ (réactif de Fenton) rentrent dans le champ d'application de la présente invention.

Pour déterminer les oxydants les plus avantageux il est possible de soumettre les oxydants à un test d'oxydation de sels de l'acide triflinique : plus faible est le rendement en anion triflate par rapport au sel d'acide triflinique consommé, meilleur est l'oxydant pour la mise en oeuvre de la présente invention ; par ailleurs il est préférable que pour une quantité stoechiométrique d'oxydant le taux de transformation (ou de consommation) de l'acide triflinique soit élevé de préférence au moins égal à 1/10 avantageusement à 1/3. On peut considérer que les oxydants donnant un rendement (RT) en sel d'acide triflique, par rapport au triflinate consommé, d'au plus 1/3, avantageusement 1/5 de préférence 1/0, sont satisfaisants. On peut signaler que certains oxydants réagissent sur l'espèce formée : par exemple $N_20_4$ donne du trifluoro nitroso methane ($CF_3N0$). Ainsi pour pouvoir utiliser de tels oxydants autrement qu'en les utilisant sur eux-mêmes il convient d'utiliser des substrats plus réactifs que lesdits oxydants. Les oxydants adaptés sont avantageusement choisis parmi le nitrate double de cérium (IV) et d'ammonium, les sels ferriques, le persulfate d'ammonium (ou de sodium) ou les hydropéroxydes notamment de tertiobutyle, ou de cyclohexyle. Entrent également dans cette famille d'oxydants les dérivés (et notamment les sels) de plomb IV tels que le tétracétate de plomb. Ils sont toutefois plus difficiles à manipuler.

Les hydroperoxydes sont avantageusement activés par des quantités catalytiques de composés du molybdène VI ($MoO_2$(acac)2 ou $Mo(CO)_6$), du vanadium V (VO $(acac)_2$), de l'osmium VIII ($OsO_4$) ou du sélénium IV ($SeO_2$).

Les persulfates, quant à eux, sont avantageusement activés par un sel métallique oxydable comme, par exemple le fer II (sel de Mohr). Un vecteur d'oxydation à base de sels cuivriques comme, par exemple, $Cu(acac)_2$ ou $(CF_3SO_3)_2Cu$, peut être ajouté en quantités catalytiques. L'oxydation peut également avoir lieu sur l'anode d'un électrolyseur, pourvu que son potentiel soit convenablement choisi. L'hydroperoxyde de tertiobutyle, liquide, est plus spécialement préféré.

Le perfluoroalcanesulfinate est employé à raison de 1 à 7 moles par mole de substrat organique, de préférence de 1 à 4 moles/mole.

Les oxydants sont utilisés à raison de 1 à 10 moles par mole de substrat, de préférence de 1 à 7 moles/mole.

Les catalyseurs sont ajoutés à raison de 0,001 à 0,5 mole par mole de substrat, de préférence 0,02 à 0,3 mole/mole.

Il a été constaté que les résultats sont meilleurs et plus reproductibles lorsque l'oxydant est introduit lentement dans le milieu réactionnel de manière continue et régulière, par exemple à l'aide d'un pousse-seringue.

La réaction est habituellement conduite dans des solvants aprotiques comme l'acétate d'éthyle ou l'acétonitrile (éventuellement additionné d'eau) mais d'autres solvants comme, par exemple, les solvants aprotiques polaires (diméthylformamide, diméthylacétamide, N-méthylpyrolidone, sulfolane, glymes, hexaméthylphosphorotriamide,...) ou l'eau sont utilisables dans la mesure où ils dissolvent le perfluoroalcanesulfinate de sodium (et, de préférence, le substrat aussi) et sont insensibles à l'action des radicaux perfluoroalkyles et des réactifs oxydants. On emploie en général de 0,5 à 30 ml de solvant par millimole de substrat, de préférence de 1 à 15 ml/mmole.

Le solvant peut être également constitué de substrat en excès. La température de réaction est habituellement comprise entre 15 et 70° C (et plus préférentiellement entre 20 et 50° C) mais des températures plus basses ou plus élevées peuvent être utilisées en fonction de la réactivité du substrat. Cependant, il est inutile de dépasser 150° C car la stabilité du perfluoroalcanesulfinate de sodium diminue au-delà de cette limite.

Les perfluoroalcanesulfinates alcalins sont des réactifs perfluoroalkylants particulièrement avantageux dans la mesure où il sont commodément obtenus par réaction entre un bromure ou un iodure de perfluoroalkyle et un système réducteur tel que le couple zinc/bioxyde de soufre (RHONE POULENC-Demande Europeenne publiée sous le N° 165.135) ou les dithionites et hydroxyméthanesulfinates alcalins (RHONE-POULENC- Demande Européenne publiée sous le N° 237.446 et 278.822, Demande Française publiée sous le N° 89/05809). En particulier, le trifluorométhanesulfinate de sodium résulte de l'action du dithionite de sodium sur le bromotrifluorométhane commercial et bon marché, employé comme gaz extincteur dans les aéroplanes et les salles d'informatique.

La méthode proposée dans le présent brevet est une méthode douce et rapide de trifluorométhylation qui permet, à l'aide d'un agent trifluorométhylant unique (souvent peu onéreux, solide, sans danger et atoxique) et d'oxydants (en particulier t-BuOOH) peu onéreux, peu toxiques et peu dangereux, de traiter un grand nombre de substrats à pression atmosphérique et température ambiante, pour conduire à une grande variété de produits d'intérêt biologique ou utiles dans la synthèse de nouveaux matériaux. En particulier, le trifluorométhanesulfinate de sodium est aisément accessible à partir de bromotrifluorométhane et de dithionite de sodium et sera prochainement disponible à l'échelle industrielle.

Cette technique est souvent complémentaire des réactions de trifluorométhylation à l'aide de CF3Br en milieu réducteur (Zn, Zn/SO2, Na2S2O4), déjà brevetées par la demanderesse, qui présentent cependant l'inconvénient de nécessiter l'usage de réacteurs sous pression, de niveaux thermiques parfois plus élevés et de substrats résistant aux conditions réductrices. Elle ne requiert pas non plus l'emploi de métaux non dissous en quantité stoechiométrique, sources d'abrasion du réacteur, comme cela est le cas lors de la trifluorométhylation des énamines par CF3Br + Zn.

Le perfluoroalcanesulfinate présente avantageusement la formule suivante(I) :

$$R\text{-}(CF2)_n - SO_2^- \qquad (I)$$

où R représente un hydrogène, un groupe alcoyle, un groupe aryle, un atome de chlore ou un atome de fluor ;
n représente un entier de 1 à 8 de préférence de 1 à 4 avec de préférence la condition que lorsque n est égal à 1, R est un fluor ou un chlore.

Le terme alcoyle est pris dans l'acception la plus large par le dictionnaire de Chimie DUVAL. Ledit alcoyle présente avantageusement au plus 15 atomes de carbone, de préférence 6.

Par aryle, on entend un radical aromatique mono ou polycyclique, condensé ou non, homo ou hétérocyclique éventuellement substitué avantageusement un radical aromatique d'au plus 15 atomes de carbone, de préférence condensé ou monocyclique, éventuellement substitué.

La présente invention vise également un procédé de perfluoroalcoylation de substrat nucléophile par mise en contact de ce dernier avec un réactif comportant :

a) un ion pertluoroalcanesulfinate, de préférence alcalin et plus préférentiellement de sodium, ou d'un perfluoroalcanesulfinate d'ammonium ou de phosphonium. (Le trifluorométhanesulfinate de sodium est plus spécialement préféré).

<u>b) un système oxydant choisi parmi les persulfates ou les hydropéroxydes, y compris le réactif de Fenton :</u>

c) un catalyseur choisi parmi les sels métalliques oxydables, les composés du molybdène VI, du vanadium V, de l'osmium VIII (ou du sélénium IV).

Des systèmes oxydants qui réagissent sur RfSO2Na suivant des processus biélectroniques conduisant à RfSO3Na, tels que H2O2 seul ou l'hypochlorite de sodium, sont exclus. Par contre, les radicaux hydroxyles engendrés par $H_2O_2$ + $Fe^{2+}$ (réactif de Fenton) rentrent dans le champ d'application de la présente invention.
La famille des substrats nucléophiles qui donnent de bons résultats est très large, ce qui est illustré par la liste établie en première page.

En ce qui concerne les dérivés aromatiques, le procédé donne des résultats intéressants pour ceux de formule (II) :

$$Ar\,(R)n \qquad (II)$$

dans laquelle :

- Ar représente un radical aromatique mono ou polycyclique, condensé ou non, homo ou hétérocyclique,
- R représente au moins un substituant choisi indépendamment parmi l'hydrogène, le chlore, le brome, les radicaux alkyles linéaires, ramifiés, cycliques saturés ou insaturés éventuellement substitués, éther, alcoxy éventuellement substitué, aryl éventuellement substitué, aryloxy, amino, hydroxy, carboxylate, acyloxy, ester, amido, nitrile, acide,
- n est un nombre entier égal à 1, 2, 3 ou 4, avantageusement
- Ar représente un radical aromatique monocyclique,
- R représente un radical donneur de préférence amino, hydroxy ou alcoxy.
  Par donneur, on entend un radical donneur par divers effets (inducteur, mésomère) dont l'effet donneur est au moins égal à un alkyle ($C_nH_{2n+1}$) léger.
  Lorsque il y a plusieurs substituants, la nucléophilie du substrat est avantageusement voisine de ou supérieure à celle du benzène.

Parmi les dérivés aromatiques de formule (II) utilisées dans le procédé de l'invention, on peut citer à titre d'enseignement par l'exemple (paradigme), et donc de façon non limitative :

- le benzène, le naphtalène, le phénanthrène, le toluène, le métaxylène, l'oxyde de phényle, le biphényle, le bromobenzène, le chlorobenzène, l'alcool benzylique, le phenyl acétate d'éthyle, la pyridine, la méthyl-2 pyridine, le pyrrole ; les amines telles que notamment l'aniline, les méthylanilines, les phénoxyanilines, l'aminonaphtalène, les diaminobenzènes, l'amino-3 pyridine ; les dérivés phénoliques parmi lesquels on peut citer les phénols, les crésols, les phénylphénols, les chlorophénols, les aminophénols, les anisoles, les méthoxyphénols, les dihydroxybenzènes, le terbutyl-4 phénol, le terbutyl-3 phénol, l'hydroxy-2 pyridine.

Avantageusement, le nombre de carbone est au plus égal à 50, de préférence à 30.
Les exemples non limitatifs suivants illustrent l'invention.

**EXEMPLES** :

**I. Perfluoroalkylation de différents substrats.**

1- Trifluorométhylation des disulfures organiques aliphatiques (primaires, secondaires ou tertiaires) et de certains disulfures aromatiques ou hétérocycliques.

Dans les exemples relatifs aux disulfures organiques, les rendements seront toujours exprimés par rapport à la stoechiométrie 1 $R_fSO_2$ -/1 RSSR.

Exemple 1 : Synthèse du (trifluorométhylthio)-3 propionate d'éthyle.

Dans un ballon tricol de 100 ml, muni d'un thermomètre, d'un réfrigérant ascendant et d'un système de coulée, sont introduits successivement, sous agitation magnétique, une solution de 1,86 g ($10.10^{-3}$ mol) de trifluorométhanesulfinate de sodium (à 83,7 % de pureté ; impureté : diméthylformamide) dans 10 ml d'eau, 0,09 g ($3.10^{-4}$ mol) de bis-acétylacétonate de molybdyle $MoO_2$ $(acac)_2$ et une solution de 1,33 g ($5.10^{-3}$ mol) de 3,3' -dithiodiproponiate d'éthyle $(SCH_2CH_2CO_2\ Et)_2$ dans 5 ml d'acétonitrile. Le mélange est maintenu à 18-20° C à l'aide d'un bain d'eau et 2,28 ml d'une solution aqueuse d'hydroperoxyde de tertiobutyle à 63 % ($15.10^{-3}$ mol) sont ajoutés en 60 mn à l'aide d'un pousse-seringue. Après la fin de la coulée, le milieu réactionnel est agité pendant 2,5 h à la température ambiante.

L'éventuel excès d'hydroperoxyde est détecté par un test colorimétrique à l'iodure de potassium et détruit par l'addition de quelques gouttes d'une solution de métabisulfite de sodium. Le mélange réactionnel est alors décanté et l'huile recueillie est extraite trois fois avec de l'éther de pétrole. Les phases éthérées sont regroupées, séchées sur sulfate de magnésium et évaporées. 1,97 g d'un liquide jaune sont ainsi obtenus. Son spectre RMN $^{19}F$, tracé en présence de (trifluorométhyl) benzène comme étalon interne, indique la formation de $5.10^{-3}$ moles de 3-(trifluorométhylthio) proponiate d'éthyle, correspondant à un rendement brut de 100 %. Une distillation "boule-à-boule" (35 à 50° C, 8,6 hectopascals (hPa)) conduit à isoler une fraction pure permettant d'accéder aux caractéristiques spectroscopiques du produit :

RMN $^{1}H$ (300 MHz, $CDCl_3$ solvant, TMS référence, $\delta$ en ppm) :
1,28 (t, J = 7 Hz, 3 H) ; 2,74 (t, J = 7 Hz, 2 H) ; 3,12 (t, J =7 Hz, 2 H) ; 4,18 (q, J = 7 Hz, 2 H)
RMN $^{19}F$ (75, 38 MHz, $CDCl_3$ solvant, $CFCl_3$ référence, $\phi_F$ en ppm) :
- 42,16 (s, 3 F)

<u>Exemples 2 à 13</u> :

On utilise, sur différents substrats, le même mode opératoire que dans l'exemple 1 sauf dans certains cas où l'hydroperoxyde de tertiobutyle est remplacé par le persulfate de potassium $K_2S_2O_8$ (additionné de sel de Mohr) ou le nitrate double de cérium (IV) et d'ammonium (CAN) et d'autres cas où l'on utilise l'acétonitrile ou l'acétate d'éthyle purs comme solvants. Les résultats sont résumés dans le tableau 1 ci-dessous :

**TABLEAU 1**

$$\underset{(1\ \text{éq.})}{R\text{-}S\text{-}S\text{-}R} + \underset{(3\ \text{éq.})}{CF_3SO_2Na} + \underset{(2\ \text{éq.})}{Ox} \xrightarrow[\text{cata}]{20°C} R\text{-}S\text{-}CF_3$$

rendement = moles $RSCF_3$/moles RSSR

| Ex | R | solv. (ml/mmol) subs. | Ox. | cata (nbre éq.) | rendement (%) |
|---|---|---|---|---|---|
| 2 | $CH_2CH_2E$ | MeCN (1) $H_2O$ (2) | t-BuOOH | - | 73* |
| 3 | d° | MeCN (3) | d° | - | 71* |
| 4 | d° | MeCN (2,5) $H_2O$ (2) | $K_2S_2O_8$ + sel Mohr | - | 64 |
| 5 **** | d° | MeCN (1) $H_2O$ (2) | CAN | - | 34* |
| 6 | $CH_2E$ | MeCN (1,6) $H_2O$ (3,3) | t-BuOOH*** | Mo VI (0,09) | 62 |
| 7 | n-$C_8H_{17}$ | MeCN (4) | d° | - | 96 |
| 8 | d° | AcOEt (4,2) | d° | - | 100 |
| 9 | c-$C_6H_{11}$ | AcOEt (3) | d° | - | 8* |
| 10 | d° | MeCN (3) | d° | - | 45* |
| 11 | t-Bu | d° | d° | | 11 |
| 12 | Hét. | MeCN (3,3) $H_2O$ (3,3) | d° | Mo VI (0,04) | 40 |
| 13 | 4-Cl-Ph | MeCN (6) $H_2O$ (2) | d° | $Mo^{VI}$ (0,06) | 32** |

$Mo^{VI}$ = $MoO_2$ $(acac)_2$

E = $CO_2Et$

Hét. = amino-5-cyano-3 (dichloro-2,6 -trifluorométhyl-4)-1 pyrazolyl-4

* rendement en produit isolé

** sous-produit : chloro-4 trifluorométhyl-x (trifluorométhylthio)-1 benzène (2 isomères dans un rapport 1/2) : rdt = 6 %

*** introduit manuellement par ampoule de coulée.

**** Comparatif

2- S-Trifluorométhylation du N,N'-bis chlorhydrate de l'ester diméthylique de la (L) cystine.

On réalise la réaction comme dans l'exemple 1, à partir de 4,4 mmol de substrat, 13,3 mmol de trifluorométhanesulfinate de sodium, 0,294 mmol de $MoO_2$ $(acac)_2$, 19,7 mmol d'hydroperoxyde de tertiobutyle et 70 ml d'eau. L'hydroperoxyde est introduit pendant 2 heures et le mélange, après repos à la température ambiante sous agitation pendant 2 heures, est extrait avec 3x30 ml d'éther de diéthyl. La phase aqueuse est alors neutralisée jusqu'à pH = 10 et extraite

à nouveau avec 3x30 ml d'éther de diéthyl. La solution éthérée est traitée avec 4,55 mmol de l'acide hydrochlorhydrique et 0,5g de N-chlorhydrate de l'ester méthylique de la S-trifluorométhyl (L)-cystéine est filtrée (47 % de rendement). Le produit est caractérisé par son spectre RMN $^1H$ ($D_2O$, TMS), (3,6 ppm (m, 2H), 3,85 ppm (s, 3H), 4,55 ppm (m, 1H), 5,65 ppm (s, 3H)), son spectre RMN $^{19}F$ (DMSO, $CFCl_3$) (s, -43,9 ppm (DMSO, $CFCl_3$)) et son $[\alpha]_D^{25}$ = + 11,69 (MeOH, c = 0,992 g/100 ml).

3- <u>S-Trifluorométhylation de l'ester diméthylique de la N,N'-diacétyle (L) cystine</u>

La réaction s'effectue comme dans l'exemple 2, à partir du 2,4 mmol de substrat, 4,82 mmol de trifluorométhanesulfonate de sodium, 7,2 mmol d'hydroperoxyde de tertiobutyle, 0,144 mmol de $MoO_2$ $(acac)_2$ et 50 mml d'acétonitrile comme solvant. Le mélange réactionnel est versé sur 100 ml d'eau et extrait avec 50+20 ml d'éther de diéthyle. L'évaporation donne 0,588 g de produit brut, lequel est soumis à la chromatographie sur silice avec un mélange acétone-éther de pétrole (40/60). 0,29 g de l'ester méthylique de la S-trifluorométhyle-N-acétyle (L) cystéine est récupérée (rendement = 46 %, pureté = 94 %).
Après recristallisation dans du cyclohexane, le produit est caractérisé par son spectre RMN $^1H$ ($D_2O$, TMS) (2,0 ppm (s, 3H), 3,4 ppm (m, 2H), 3,85 ppm (s, 3H), 4,95 ppm (m, 1H), 6,75 ppm (m,1H), son spectre RMN $^{19}F$ ($D_2O$, $CFCl_3$) (s, -41,1 ppm (DMSO, $CFCl_3$)) et son $[\alpha]_D^{25}$ = + 41,15 (MeOH, c = 1,152 g/ 100 ml).

4- <u>S-Trifluorométhylation du N, N'-dichlorhydrate de l'ester diméthylique de la (D.L)-homocystine</u>

Les conditions de l'exemple 2 sont appliquées à 2,7 mmol de substrat, 5,4 mmol de trifluorométhanesulfinate de sodium, 8,12 mmol d'hydroperoxyde de tertiobutyle, 0,162 mmol de $MoO_2$ $(acac)_2$ et 60 ml d'eau comme solvant.

Après la mise en oeuvre habituelle, 0,38 g de pure chlorhydrate de l'ester méthylique de la S-trifluorométhylation de la (D.L)-homocystine est récupéré et caractérisé par son spectre RMN $^1H$ ($D_2O$, TMS) (2,45 ppm (m, 2H), 3,15 ppm (m, 2H), 3,85 ppm (s, 3H), 4,3 ppm (m, 1H), 4,80 ppm (s, 3H)) et son spectre RMN $^{19}F$ ($D_2O$, $CFCl_3$) (s, -4,8 ppm).

5- <u>S-Trifluorométhylation de l'ester diméthylique de la N,N'-diacétyl -(D,L) homocystine</u>

Les conditions de l'exemple 3 sont appliquées à 2,34 mmol de substrat, 4,68 mmol de trifluorométhanesulfinate de sodium, 7,23 mmol d'hydropéroxyde de tertiobutyl, 0,14 mmol de $MoO_2$ $(acac)_2$ et 60 ml d'acétonitrile. La chromatographie est effectuée avec un mélange d'acétone-éther de pétrole de 50/50. 90 % d'ester méthylique de la S-trifluorométhyle-N-acétyle de la (D,L)-homocystéine pure est récupéré (rendement = 37 %).

RMN $^1H$ ($CDCl_3$, TMS) : 1,9 ppm (s, 3H), 2,1 ppm (m, 2H), 2,85 (m, 2H), 3,65 ppm (s, 3H), 4,65 ppm (m, 1H), 7,0 ppm (m, 1H).
RMN $^{19}F$ ($CDCl_3$, $CFCl_3$) : -42,1 ppm (s).

6- <u>Sulfure de trifluorométhyle et de n-octyle à partir du disulfure de nitro-4 phényle et de n-octyle</u>

Dans les conditions de l'exemple 7.2, 3,13 mmol de sulfure de n-octyle de nitro-4 phényle et 6,25 mmol de trifluorométhanesulfinate de sodium (dans 20 ml d'acétonitrile) sont traités avec 9,39 mmol d'hydroperoxyde de tertiobutyle. Le sulfure de trifluorométhyle de n-octyl, caractérisé par son spectre RMN $^{19}F$ et $^1H$, est isolé après chromatographie sur silice avec de l'éther de pétrole (rendement = 22 %).

7- <u>Trifluorométhylation du disulfure de diphényle par le trifluorométhanesulfinate de sodium en milieu eau-acétonitrile et en présence d'hydroperoxyde de tertiobutyle : exemple 14</u>.

**Exemple 7.1**

On utilise le même mode opératoire que dans l'exemple 1, à l'exception de l'ajout de catalyseur au molyodène. Les quantités de départ sont :

- 5 mmol de disulfure de diphényl,
- 10 mmol de trifluorométhanesulfinate de sodium,
- 10 ml d'eau,
- 5 ml d'acétonitrile,
- 15 mmol d'hydroperoxyde de tertiobutyl.

Après la mise en oeuvre habituelle, le mélange de disulfures de diphényle trifluorométhylés (I) (3 isomères) et de sulfure (II) de phényle de trifluorométhyle est obtenu avec un rapport (I)/(II) = 40/60 et rendement total de 12 %.

**Exemple 7.2**

La réaction s'effectue comme dans l'exemple 7.1, à l'exception que l'acétonitrile pure est utilisé comme solvant (4 ml/mmol de substrat). (I) (2 isomères) et (II) sont obtenus dans un rapport de 64/36 dont le rendement total est de 14 %.

**Exemple 7.3**

La réaction s'effectue comme dans l'exemple 7.1 à l'exception que l'acétate d'éthyle pur est utilisé comme un solvant (4 ml/mmol de substrat). (I) (3 isomères) et (II) sont obtenus dans un rapport de 35/65 et de 34 % de rendement total.

8- Trifluorométhylation du disulfure de dichloro-4,4'-diphényle par le trifluorométhanesulfinate de sodium en milieu acétate d'éthyle et en présence d'hydroperoxyde de tertiobutyle : exemple 15.

Dans un ballon tricol muni d'un thermomètre, d'un réfrigérant ascendant et d'une ampoule de coulée équipée d'un robinet à pointeau, sont introduits successivement sous agitation magnétique :

- 1,87 g ($12,10^{-3}$ mol) de trifluorométhanesulfinate de sodium en solution à 4,6 % dans l'acétate d'éthyle,
- 0,09 g ($3.10^{-4}$ mol) de $MoO_2$ $(acac)_2$,
- 1,43 g ($5.10^{-3}$ mol) de disulfure de dichloro-4,4′-diphényle dissous dans 40 ml d'acétate d'éthyle.

Le mélange est maintenu à 18-20° C et 2,28 ml d'une solution aqueuse d'hydroperoxyde de tertiobutyle à 63 % ($15.10^{-3}$ mol) sont additionnés en 30 minutes. Le milieu réactionnel est abandonné 2,5 h à température ambiante puis évaporé. Le résidu ainsi obtenu est extrait par un mélange eau-éther de pétrole (50/50) puis trois fois à l'éther de pétrole pur. Les phases éthérées, regroupées, séchées sur sulfate de magnésium et évaporées, fournissent un solide jaune contenant du disulfure de départ et du 4-chloro (trifluorométhylthio) benzène correspondant à un rendement de 2 %. Le résidu insoluble dans l'eau et l'éther de pétrole est constitué de chloro-4 -(trifluorométhyl)-2 thiophénol représentant un rendement de 30 %.

9- Trifluorométhylation du n-octanethiol

On emploie le même mode opératoire que dans l'exemple 1, les constituants réactionnels étant mis en oeuvre dans les proportions suivantes :

- n-octanethiol : 1 éq.
- hydroperoxyde de tertiobutyle : 1,5 éq.
- trifluorométhanesulfinate de sodium : 1 éq.
- acétonitrile : 0,5 ml/mmole de substrat.
- eau : 1 ml/mmole de substrat.

On obtient le trifluorométhyl n-octyl thioéther avec un rendement de 12 %.

10- Trifluorométhylation des composés aromatiques

Les composés aromatiques ont été trifluorométhylés par le trifluorométhanesulfinate de sodium en présence d'hydroperoxyde de tertiobutyle et de trifluorométhanesulfonate cuivrique en quantités catalytiques, suivant les conditions générales décrites dans l'exemple 1:

**10.1. Trifluorométhylation des phénols et de leurs dérivés**

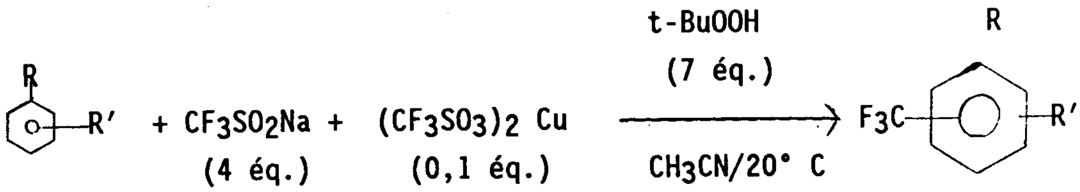


| Ex | R | R' | MeCN (ml/mmol subs.) | Durée coulée t-BuOOH | Rendement |
|---|---|---|---|---|---|
| 17 | OH | H | 3 | 4,5 h | 2-$CF_3$ = 16 %<br>3-$CF_3$ = 4,6 %<br>4-$CF_3$ = 24 % |
| 18 | 1-OMe | 3-OMe | 4 | 3 h | 2-$CF_3$ = 15 %<br>6-$CF_3$ = 59 % |

Les produits de trifluorométhylation du phénol ont été identifiés dans le mélange réactionnel brut par comparaison de leurs spectres de RMN$^1$H et $^{19}$F avec ceux des trifluorométhyl-phénols isomères commerciaux et quantifiés par RMN $^{19}$F en présence de (trifluorométhyl) benzène comme étalon interne. Les produits de trifluorométhylation du 1,3-diméthoxybenzène ont été séparés, isolés et caractérisés spectroscopiquement et par leur analyse élémentaire.

11- Trifluorométhylation de l'hydroxy-4 benzoate de méthyle

Lorsque l'on fait réagir le substrat dans les conditions décrites dans l'exemple ci-dessus (17), on obtient le trifluorométhyl-3 hydroxy-4 benzoate de méthyle avec un rendement de 19 %, après chromatographie sur silice avec un mélange de diéthyl-éther de pétrole de 30 sur 70.

RMN $^1$H ($CDCl_3$, TMS) : 3,9 ppm (s, 3H), 7,0 ppm (d, 1H, J= 8 Hz), 8,0 ppm (dd, 1H), 8,15 ppm (d, 1H, J= 2 Hz), 9,7 ppm (m, 1H) $^{19}$F RMN ($CDCl_3$, $CFCl_3$) : -63,3 ppm.

12- Trifluorométhylation de l'acétanilide

1,38 g (10 mmol) d'acétanilide, 6,56 g (40 mmol) de trifluorométhanesulfinate de sodium, 0,3615 g (1 mmol) de trifluorométhanesulfonate cuivrique, 20 ml d'acétonitrile et 40 ml d'eau sont placés dans un tricol équipé d'un thermomètre, d'un réfrigérant à reflux et d'un agitateur magnétique. 10,64 ml d'une solution aqueuse de 63 % d'hydropéroxyde de tertiobutyle (70 mmol) sont introduits, au moyen d'une seringue à 18-20°C pendant 4 h 40 min. Le mélange réactionnel est alors soumis au repos à une température ambiante pendant 2,5 heures. L'excès d'hydroperoxyde, détecté par le test des iodures est détruit avec du métabisulfite de sodium aqueux.
Après dilution avec 40 ml d'eau, le milieu est extrait avec 3x40 ml d'éther de diéthyle. La solution éthérée est évaporée. Le résidu est examiné par RMN $^{19}$F ( $CDCl_3$, $CFCl_3$) en présence d'un trifluoro-2,2,2-éthanol comme étalon interne. Les spectres sont comparés avec ceux des échantillons authentiques d'acétanilides trifluorométhylés préparés par acétylation des trifluorométhyle aniline commercial, ortho, métha et para.
On en déduit les rendement suivants :

| | | |
|---|---|---|
| - $CF_3$-2 acétanilide, | Rendement = 30 % | -61,32 ppm |
| - $CF_3$-3 acétanilide, | Rendement = 7 % | -63,07 ppm |

(suite)

| | | |
|---|---|---|
| - $CF_3$-4 acétanilide, | Rendement = 14,5 % | -62,43 ppm |

13- Trifluorométhylation de l'aniline

Ce substrat est soumis aux conditions décrites dans l'exemple 12 (même procédé, mêmes quantités, mêmes analyses). Le spectre RMN $^{19}F$ indiques la formation de 2 isomères de trifluorométhylanilines (-61,25 et -63,85 ppm) avec un rendement total de 10 %.

14- Trifluorométhylation de dichloro-2,6-aniline

Ce substrat est traité dans les mêmes conditions que l'acétanilide (exemple 12) ($Ar-NH_2/CF_3SO_2Na/(CF_3SO_3)_2$ Cu/t-BuOOH = 1/4/0,1/7), soit dans un mélange eau-acétonitrile (4 et 2 ml par mmole de substrat) ou de l'acétonitrile pure (6 ml par mmol de substrat). On obtient 2 isomères de la dichloro-2,6-anilines trifluorométhylés caractérisés par leur spectre RMN $^{19}F$ ($CDCl_3$, $CFCl_3$). Leurs rendements sont dépendants de la nature du solvant :

| Solvant | (I) (-61,97 ppm) | (II) (-63,08 ppm) |
|---|---|---|
| MeCN - $H_2O$ | 11 % | 4 % |
| MeCN | 22 % | 7 % |

15- Trifluorométhylation de la N-acétyle pyrrole.

La N-acétyle pyrrole, dissous dans de l'acétonitrile pure (6 mml par mmol) est soumis aux mêmes conditions que l'acétanilide (substrat/$CF_3SO_2Na/(CF_3SO_3)2$ Cu/t-BuOOH = 1/4/0,1/7).
Le résidu est purifié par chromatographie sur silice avec le mélange diéthyle-éther de pétrole (20/80). Le trifluorométhyl-2 N-acétyl pyrrole pur est isolé avec un rendement de 35 %.

RMN $^1H$ ($CDCl_3$, TMS) : 2,6 ppm (s, 3H), 6,3 ppm (m, 1H), 6,9 ppm (m, 1H), 7,4 ppm (m, 1H).
RMN $^{19}F$ ($CDCl_3$, $CFCl_3$) : -59,42 ppm (s).

16- Trifluorométhylation du diméthoxy-1,3 benzène en l'absence d'espèces cuivriques

On fait réagir du diméthoxy-1,3-benzène dans les mêmes conditions rapportées à l'exemple 10-1 (18), excepté la présence du trifluorométhanesulfonate cuivrique. Quatre diméthoxy-1,3-benzènes trifluorométhylés sont détectés par RMN $^{19}F$ :

| | | |
|---|---|---|
| - $CF_3$-4-1,3- $(OMe)_2$-$C_6H_3$ | (I) | Rendement = 20 % |
| - $CF_3$-2-1,3- $(OMe)_2$-$C_6H_3$ | (II) | Rendement = 7,5 % |
| - $(CF_3)_2$-4,6 -1,3-$(OMe)_2$-$C_6H_2$ | (III) | Rendement = 23 % |
| - $(CF_3)_2$-2,6 -1,3-$(OMe)_2$-$C_6H_2$ | (IV) | Rendement = 7,5 % |

Le mélange de (I) + (II) + (IV) est isolé du résidu de la réaction par chromatographie sur silice ($Et_2O$/éther de pétrole = 3/247) et (III) est élué sous forme pure avec $Et_2O$/ éther de pétrole = 5/245. (I), (II) et (III) sont alors séparés par chromatographie préparative (Carbowax 20M sur Chromosorb WAW DMS, 3/8" 3 mètres). Tous ces composés isolés ont été analysés par RMN $^1H$, RMN $^{19}F$ et par spectrométrie de masse.

$^1H$ RMN ($CDCl_3$, TMS) :

**(I)** : 7,470 ppm (d, 1H, J= 8,5 Hz), 6,497-7,255 ppm (m, 2H), 3,867 ppm (s, 3H), 3,836 ppm (s, 3H).

**(II)** : 7,384 ppm (t, 1H, J = 8,5 Hz), 6,611 ppm (d, 2H, J = 8,5 Hz), 3,862 ppm (s, 6H).
**(III)** : 7,7 ppm (s, 1H), 6,5 ppm (s, 1H), 3,9 ppm (s, 6H).
**(IV)** : 7,722 ppm (d, 1H, J = 8,5 Hz), 6,829 ppm (d, 1H, J= 9,0 Hz), 3,942 ppm (s, 3H), 3,890 ppm (s, 3H).

RMN $^{19}F$ ($CDCl_3$, $CFCl_3$) :

(I) : -61,974 ppm (s)
(II) : -55,498 ppm (s)
(III) : -62,3 ppm (s)
(IV) : -57,766 ppm (s), - 60,939 ppm (s).

17- <u>Trifluorométhylation des acétates d'énols</u>

L'acétate d'isopropényle et l'acétate d'énol correspondant à la undécan-6-one ont été traités par le trifluorométhanesulfinate de sodium et l'hydroperoxyde de tertiobutyle, au sein de l'acétonitrile et en présence de quantités catalytiques de trifluorométhanesulfonate cuivrique, de la même manière que les composés aromatiques ci-dessus.

18- <u>Trifluorométhylation de l'acétate d'isopropényle</u>

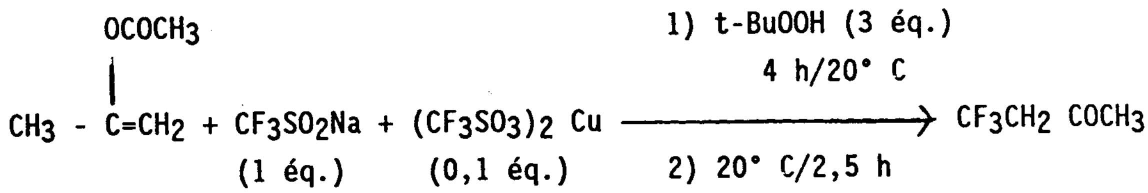


Le solvant acétonitrile est utilisé à raison de 1,5 ml/mmole d'acétate d'isopropényle.
Les spectres RMN $^{1}H$ et $^{19}F$ du mélange réactionnel final, en présence de (trifluorométhyl) benzène comme étalon interne, révèlent la formation de 4,4,4-trifluorobutan-2-one avec un rendement de 53 %. Ce produit, relativement volatil, a été isolé sous forme de la (2,4-dinitrophényl) hydrazone pure correspondante, qui a été caractérisée par ses spectres RMN $^{1}H$ et $^{19}F$, son spectre de masse ainsi que par son analyse élémentaire.

19- <u>Trifluorométhylation de l'acétate d'énol de la undécan-6-one</u>.

On opère de la même manière que dans l'exemple 19 à partir de 4 équivalents d'hydroperoxyde de tertiobutyle, 2 équivalents de trifluorométhanesulfinate de sodium, 0,25 équivalents de trifluorométhanesulfonate cuivrique et 3 ml d'acétonitrile par mmole de substrat. La durée d'addition de la solution aqueuse d'hydroperoxyde à 63 % est de 2,75 h.
Après le traitement usuel, on isole un mélange de 5-(trifluorométhyl) undécan-6-one racémique (rendement = 3 %) et de deux isomères E et Z du 6-acétoxy-7-(trifluorométhyl) undéc-5-ène (rdts = 23 % et 2 %). Après hydrolyse, ce mélange fournit la 5-(trifluorométhyl) undécan-6-one pure.

20- <u>Trifluorométhylation de l'acétate d'énol de la undécan-6-one en présence d'acétate de sodium</u>.

On opère de la même manière que dans l'exemple 20 à partir de 2 équivalents d'hydroperoxyde de tertiobutyle, 4 équivalents de trifluorométhanesulfinate de sodium, 0,25 équivalents de trifluorométhanesulfonate cuivrique, 1 équivalent d'acétate de sodium et 3 ml d'acétonitrile par mmole de substrat.
Après le traitement usuel, on isole un mélange équimoléculaire des isomères E et Z du acétoxy-6 (trifluorométhyl)-7 undéc-5-ène.

21- Trifluorométhylation de la hydroxy-4 (méthyl)-6 pyran-2-one.

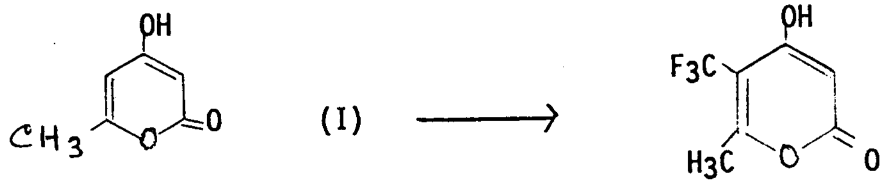

(I)

(II)

On opère de la même façon que dans l'exemple 19 à partir de 4 équivalents de trifluorométhanesulfinate de sodium, 7 équivalents d'hydroperoxyde de tertiobutyle, 0,1 équivalents de trifluorométhanesulfonate cuivrique et 4 ml d'acétonitrile par mmole de substrat. La durée de coulée de l'hydroperoxyde de tertiobutyle est de 3,75 h. Après traitement usuel, on recueille le produit (II), isomériquement pur, sous forme d'un solide caractérisé par ses spectres RMN $^1H$ et $^{19}F$, son spectre de masse ainsi que par son analyse élémentaire. Le rendement en produit isolé est de 45 %.

**II Test pour le choix des oxydants**

Divers exemples d'oxydants ont été engagés ci-dessous pour démontrer la valeur du test pour choisir les oxydants.

**II. 4. Oxydation par les hydropéroxydes**

Ces opérations ont été effectuées dans un ballon tricol de 250 ml avec agitation centrale, surmonté d'une ampoule de coulée, d'une gaine thermométrique et d'un réfrigérant ascendant. Nous les résumons dans le tableau :

| SOLVANT | OXYDANT | CATALYSEURS | T°C | DUREE ESSAI (h) | TT CF3SO2$^-$ (%) | RT CF3SO3$^-$ (%) |
|---|---|---|---|---|---|---|
| Acétone | HPOC | VO(AcAc)2 | 25°-50° | 6 | 95 | 8 |
| Ac. d'éthyle | HPOCH | MoO2(AcAc)2 | 0°- 5° | 4 | 100 | 0 |
| Eau | t.BuOOH | MoO2(AcAc)2 | 0°- 5° | 2 | 91,5 | 15,3 |
| Ac.d'éthyle | t.BuOOH | MoO2(AcAc)2 | 25° | 2 | 100 | 0,1 |
| HPOC = hydropéroxyde de cumyle ; HPOCH = hydropéroxyde de cyclohexyle | | | | | | |

Les résultats indiquent essentiellement la coupure prépondérante de la liaison carbone - soufre (RT > 90 %). Dans ces essais nous retrouvons essentiellement, parmi les sels résiduaires, de l'hydrogénosulfite de sodium et relativement peu de fluorure.

**II. 5. Oxydation par l'eau de Javel ou l'eau oxygénée (comparatif).**

On opère dans le même appareillage que celui décrit dans le paragraphe précédent.

Lors de l'essai avec l'eau de Javel, on utilise un large excès de celle-ci (eau de Javel à 15 % de chlore actif). La réaction est peu exothermique : la température passe de 22,4° à 28,3°C en 1 heure. On dose le milieu final par chromatographie ionique : TT CF3SO2Na = 43,4 %, RT CF3SO3Na = 91 %.

Avec l'eau oxygénée à 30 % on utilise 6 fois la stoechiométrie par rapport à CF3SO2Na. La réaction est très exothermique. Après 6 heures de réaction entre 0°C et 25°C, le taux de transformation de CF3SO2Na est de 100 % et le rendement (RR) en CF3SO3Na de 96 %. On ne détecte pas de fluorure.

Dans ce cas, la coupure homolytique ne se fait pas. Il y a transfert de matière en faveur du triflinate pour donner du triflate.

Dans ces deux derniers cas, la coupure homolytique ou hétérolytique de la liaison carbone-soufre ne se produit pas.

## Revendications

1. Réactif de perfluoroalcoylation comportant :

   a) un ion pertluoroalcanesulfinate ;

   b) un système oxydant choisi parmi les persulfates ou les hydropéroxydes, y compris le réactif de Fenton ;

   c) un catalyseur choisi parmi les sels métalliques oxydables, les composés du molybdène VI, du vanadium V, de l'osmium VIII ou du sélénium IV.

2. Réactif selon la revendication 1, caractérisé par le fait que ledit système oxydant est choisi parmi les persulfates et par le fait que le catalyseur est choisi parmi les sels métalliques oxydables.

3. Réactif selon les revendications 1 et 2, caractérisé par le fait que ledit système oxydant est choisi parmi les persulfates d'ammonium et de sodium.

4. Réactif selon les revendications 1 à 3, caractérisé par le fait que ledit catalyseur est choisi parmi les vecteur d'oxydation à base de sels cuivriques avantageusement $Cu(acac)_2$ ou $(CF_3SO_3)_2Cu$.

5. Réactif selon les revendications 1 à 4, caractérisé par le fait que ledit système oxydant est choisi parmi ceux qui, utilisés pour oxyder les ions trifliniques, donnent un rendement de transformation en triflate d'au plus 1/3, avantageusement d'au plus 1/10.

6. Réactif selon les revendications 1 à 5, caractérisé par le fait que ledit système oxydant est choisi parmi, les persulfates d'ammonium ou de sodium ou les hydroxypéroxydes notamment de tertioalkyle ou de cycloalkyle, de préférence de tertiobutyle ou de cyclohexyle.

7. Réactif selon les revendications 1 à 6, caractérisé par le fait que ledit perfluoroalcane sulfinate présente avantageusement la formule suivante (I) :

   $$R\text{-}(CF2)n - SO2^- \qquad (I)$$

   ⇨ où R représente un hydrogène, un groupe alcoyle, un groupe aryle, un atome de chlore ou un atome de fluor ;

   ⇨ n représente un entier de 1 à 8 de préférence de 1 à 4 avec de préférence la condition que lorsque n est égal à 1, R est un fluor ou un chlore.

8. Procédé de perfluoroalkylation de substrat nucléophile par mise en contact de ce dernier avec un réactif comportant :

   a) un on perfluoroalcanesulfinate ;

   b) un système oxydant choisi parmi les persulfates d'ammonium et de sodium ou les hydropéroxydes, y compris le réactif de Fenton ;

   c) un catalyseur choisi parmi les sels métalliques oxydables, les composés du molybdène VI, du vanadium V, de l'osmium VIII ou du sélénium IV.

9. Procédé selon la revendication 8, caractérisé par le fait que ledit système oxydant est choisi parmi les persulfates et par le fait que le catalyseur est choisi parmi les sels métalliques oxydable.

10. Procédé selon l'une des revendications 8 et 9, caractérisé par le fait queledit système oxydant est choisi parmi les persulfates d'ammonium et de sodium.

11. Procédé selon l'une des revendications 8 à 10, caractérisé par le fait que ledit système oxydant est choisi parmi ceux qui, utilisés pour oxyder les ions trifliniques, donnent un rendement de transformation en triflate d'au plus 1/3.

12. Procédé selon la revendication 11, caractérisé par le fait que ledit rendement de transformation est d'au plus 1/10.

13. Procédé selon l'une des revendications 8 à 12, caractérisé par le fait que ledit système oxydant est choisi parmi, les persulfates d'ammonium ou de sodium ou les hydroxypéroxydes notamment de tertioalkyle ou de cycloalkyle, de préférence de tertiobutyle ou de cyclohexyle.

14. Procédé selon l'une des revendications 8 à 13, caractérisé par le fait que ledit le perfluoroalcanesulfinate est employé à raison de 1 à 7 moles par mole de substrat organique, de préférence de 1 à 4 moles/mole.

15. Procédé selon l'une des revendications 8 à 14, caractérisé par le fait que ledit oxydant est utilisé à raison de 1 à 10 moles par mole de substrat, de préférence de 1 à 7 moles/mole.

16. Procédé selon l'une des revendications 8 à 15, caractérisé par le fait que lesdits catalyseurs sont ajoutés à raison de 0,001 à 0,5 mole par mole de substrat, de préférence 0,02 à 0,3 mole/mole.

17. Procédé selon l'une des revendications 8 à 16, caractérisé par le fait que ledit oxydant est introduit lentement dans le milieu réactionnel de manière continue et régulière.

18. Procédé selon l'une des revendications 8 à 17, caractérisé par le fait que ledit substrat nucléophile est choisi parmi :

⇨ les sulfures et disulfures organiques aliphatiques (primaires, secondaires ou tertiaires), aromatiques ou hétérocycliques,
⇨ les thiols,
⇨ les oléfines et les acétyléniques éventuellement activés par des groupements électrodonneurs, comme, par exemple, les énols et leurs dérivés (en particulier éthers et esters d'énols), les énamines et leurs dérivés, les éthers acétyléniques, les ynamines,...,
⇨ les acrylamides,
⇨ les cétones énolisables (β-dicétones, β-cétoesters),
⇨ les cétones ou dérivés d'acides α, β-insaturés, éventuellement cyliques comme, par exemple, l'uracile ou l'uridine,
⇨ les dérivés homo ou hétéroaromatiques, mono ou polycycliques, condensés ou non, substitués de préférence par au moins un groupe électrodonneur par induction et/ou mésomérie comme, par exemple les halogénobenzènes, les phénols, les anilines, les anisoles, le thiophène et dérivés.

19. Procédé selon l'une des revendications 8 à 18, caractérisé par le fait que le nombre de carbone dudit substrat nucléophile est au plus égal à 50, de préférence à 30.

20. Procédé selon l'une des revendications 8 à 19, caractérisé par le fait que ledit perfluoroalcane sulfinate présente avantageusement la formule suivante (I) :

$$R\text{-}(CF2)n - SO2^{-} \qquad (I)$$

⇨où R représente un hydrogène, un groupe alcoyle, un groupe aryle, un atome de chlore ou un atome de fluor ;
⇨où n représente un entier de 1 à 8 de préférence de 1 à 4 avec de préférence la condition que lorsque n est égal à 1, R est un fluor ou un chlore.

**Claims**

1. Perfluoroalkylation reagent containing:

a) a perfluoroalkanesulphinate ion;
b) an oxidizing system chosen from persulphates and hydroperoxides, including Fenton's reagent;
c) a catalyst chosen from oxidizable metal salts, molybdenum VI, vanadium V, osmium VIII or selenium IV compounds.

2. Reagent according to Claim 1, characterized in that the said oxidizing system is chosen from persulphates and in

that the catalyst is chosen from oxidizable metal salts.

**3.** Reagent according to Claims 1 and 2, characterized in that the said oxidizing system is chosen from ammonium persulphate and sodium persulphate.

**4.** Reagent according to Claims 1 to 3, characterized in that the said catalyst is chosen from oxidation vector based on cupric salts, advantageously $Cu(acac)_2$ or $(CF_3SO_3)_2Cu$.

**5.** Reagent according to Claims 1 to 4, characterized in that the said oxidizing system is chosen from those which, when used to oxidize triflinic ions, give a yield for conversion into triflate of at most 1/3, advantageously of at most 1/10.

**6.** Reagent according to Claims 1 to 5, characterized in that the said oxidizing system is chosen from ammonium persulphate, sodium persulphate and hydroxyperoxides, in particular tert-alkyl or cycloalkyl hydroxyperoxide, preferably tert-butyl or cyclohexyl hydroxyperoxide.

**7.** Reagent according to Claims 1 to 6, characterized in that the said perfluoroalkane sulphinate advantageously has the formula (I) below:

$$R\text{-}(CF2)n\text{-}SO2^- \qquad (I)$$

- in which R represents a hydrogen, an alkyl group, an aryl group, a chlorine atom or a fluorine atom;
- n represents an integer from 1 to 8, preferably from 1 to 4, preferably with the condition that when n is equal to 1, R is a fluorine or a chlorine.

**8.** Process for the perfluoroalkylation of a nucleophilic substrate by placing this substrate in contact with a reagent containing:

a) a perfluoroalkanesulphinate ion;
b) an oxidizing system chosen from ammonium persulphate and sodium persulphate and hydroperoxides, including Fenton's reagent;
c) a catalyst chosen from oxidizable metal salts, molybdenum VI, vanadium V, osmium VIII or selenium IV compounds.

**9.** Process according to Claim 8, characterized in that the said oxidizing system is chosen from persulphates and in that the catalyst is chosen from oxidizable metal salts.

**10.** Process according to either of Claims 8 and 9, characterized in that the said oxidizing system is chosen from ammonium persulphate and sodium persulphate.

**11.** Process according to one of Claims 8 to 10, characterized in that the said oxidizing system is chosen from those which, when used to oxidize triflinic ions, give a yield for conversion into triflate of at most 1/3.

**12.** Process according to Claim 11, characterized in that the said conversion yield is at most 1/10.

**13.** Process according to one of Claims 8 to 12, characterized in that the said oxidizing system is chosen from ammonium persulphate, sbdium persulphate and hydroxyperoxides, in particular tert-alkyl or cycloalkyl hydroxyperoxide, preferably tert-butyl or cyclohexyl hydroxyperoxide.

**14.** Process according to one of Claims 8 to 13, characterized in that the said the perfluoroalkanesulphinate is used in a proportion of from 1 to 7 mol per mole of organic substrate, preferably from 1 to 4 mol/mole.

**15.** Process according to one of Claims 8 to 14, characterized in that the said oxidizing agent is used in a proportion of from 1 to 10 mol per mole of substrate, preferably from 1 to 7 mol/mole.

**16.** Process according to one of Claims 8 to 15, characterized in that the said catalysts are added in a proportion of from 0.001 to 0.5 mol per mole of substrate, preferably 0.02 to 0.3 mol/mole.

17. Process according to one of Claims 8 to 16, characterized in that the said oxidizing agent is introduced slowly, continuously and uniformly into the reaction medium.

18. Process according to one of Claims 8 to 17, characterized in that the said nucleophilic substrate is chosen from:

- aliphatic (primary, secondary or tertiary aliphatic), aromatic or heterocyclic organic sulphides and disulphides,
- thiols,
- olefins and acetylenes optionally activated with electron-donating groups such as, for example, enols and derivatives thereof (in particular enol ethers and enol esters), enamines and derivatives thereof, acetylenic ethers, ynamines, etc.,
- acrylamides,
- enolizable ketones (β-diketones, β-keto esters),
- ketones or α,β-unsaturated acid derivatives, which are optionally cyclic, such as, for example, uracil or uridine,
- fused or non-fused, mono- or polycyclic, homo- or heteroaromatic derivatives, preferably substituted with at least one electron-donating group by induction and/or mesomerism, such as, for example, halobenzenes, phenols, anilines, anisoles, thiophene and derivatives.

19. Process according to one of Claims 8 to 18, characterized in that the carbon number of the said nucleophilic substrate is not more than 50, preferably not more than 30.

20. Process according to one of Claims 8 to 19, characterized in that the said perfluoroalkanesulphinate advantageously has the formula (I) below:

$$R\text{-}(CF2)n\text{-}SO2^- \qquad (I)$$

- in which R represents a hydrogen, an alkyl group, an aryl group, a chlorine atom or a fluorine atom;
- in which n represents an integer from 1 to 8, preferably from 1 to 4, preferably with the condition that when n is equal to 1, R is a fluorine or a chlorine.

**Patentansprüche**

1. Reagenz zur Perfluoralkylierung, enthaltend:

a) ein Perfluoralkansulfination;
b) ein Oxidationssystem, ausgewählt aus Persulfaten oder Hydroperoxiden einschließlich eines Fenton-Reagenzes;
c) einen Katalysator, ausgewählt aus oxidierbaren Metallsalzen, Molybdän(VI)-Verbindungen, Vanadium(V)-Verbindungen, Osmium(VIII)-Verbindungen und Selen(IV)-Verbindungen.

2. Reagenz nach Anspruch 1, dadurch gekennzeichnet, daß das Oxidationssystem ausgewählt ist aus Persulfaten und daß der Katalysator ausgewählt ist aus oxidierbaren Metallsalzen.

3. Reagenz nach Anspruch 1 oder 2, dadurch gekennzeichnet, daß das oxidierende System (Oxidationssystem) ausgewählt ist aus Ammonium- und Natriumpersulfat.

4. Reagenz nach einem der Ansprüche 1 bis 3, dadurch gekennzeichnet, daß der Katalysator ausgewählt ist aus Oxidationsträgern auf Basis von Kupfersalzen, vorzugsweise $Cu(acac)_2$ oder $(CF_3SO_3)_2Cu$.

5. Reagenz nach einem der Ansprüche 1 bis 4, dadurch gekennzeichnet, daß das Oxidationssystem aus solchen Systemen ausgewählt ist, welche, wenn sie zur Oxidation von Trifluormethansulfinationen verwendet werden, eine Ausbeute bei der Umsetzung zu Triflat von höchstens 1/3, vorzugsweise von höchsten 1/10, liefern.

6. Reagenz nach einem der Ansprüche 1 bis 5, dadurch gekennzeichnet, daß das Oxidationssystem ausgewählt ist aus Ammonium- oder Natriumpersulfat oder insbesondere Tertioalkyl- oder Cycloalkylhydroperoxiden, vorzugsweise Tertiobutyl- oder Cyclohexylhydroxyperoxid.

7. Reagenz nach einem der Ansprüche 1 bis 6, dadurch gekennzeichnet, daß das Perfluoralkansulfinat vorzugsweise die folgende Formel (I) aufweist

$$R\text{-}(CF_2)_n\text{-}SO_2^- \qquad (I)$$

in der:

- R Wasserstoff, eine Alkylgruppe, eine Arylgruppe, ein Chloratom oder ein Fluoratom darstellt;
- n eine ganze Zahl von 1 bis 8, vorzugsweise von 1 bis 4, darstellt, vorzugsweise mit der Maßgabe, daß R Fluor oder Chlor ist, wenn n gleich 1 ist.

**8.** Verfahren zur Perfluoralkylierung eines nucleophilen Ausgangsstoffes durch Inkontaktbringen des letzteren mit einem Reagenz, enthaltend:

a) ein Perfluoralkansulfination;
b) ein Oxidationssystem, ausgewählt aus Persulfaten oder Hydroperoxiden einschließlich eines Fenton-Reagenzes;
c) einen Katalysator, ausgewählt aus oxidierbaren Metallsalzen, Molybdän(VI)-Verbindungen, Vanadium(V)-Verbindungen, Osmium(VIII)-Verbindungen oder Selen(IV)-Verbindungen.

**9.** Verfahren nach Anspruch 8, dadurch gekennzeichnet, daß das oxidierende System (Oxidationssystem) ausgewählt ist aus Persulfaten und daß der Katalysator ausgewählt ist aus oxidierbaren Metallsalzen.

**10.** Verfahren nach Anspruch 8 oder 9, dadurch gekennzeichnet, daß das Oxidationssystem ausgewählt ist aus Ammonium- und Natriumpersulfat.

**11.** Verfahren nach einem der Ansprüche 8 bis 10, dadurch gekennzeichnet, daß das Oxidationssystem aus solchen Systemen ausgewählt ist, welche, wenn sie zur Oxidation von Trifluormethansulfinationen verwendet werden, eine Ausbeute bezuglich der Umsetzung zu Triflat von höchstens 1/3 liefern.

**12.** Verfahren nach Anspruch 11, dadurch gekennzeichnet, daß die Ausbeute der Umsetzung höchstens 1/10 beträgt.

**13.** Verfahren nach einem der Ansprüche 8 bis 12, dadurch gekennzeichnet, daß das Oxidationssystem ausgewählt ist aus Ammonium- oder Natriumpersulfat und insbesondere Tertioalkyl- oder Cycloalkylhydroxyperoxiden, vorzugsweise Tertiobutyl- oder Cyclohexylhydroxyperoxid.

**14.** Verfahren nach einem der Ansprüche 8 bis 13, dadurch gekennzeichnet, daß das Perfluoralkansulfinat in Mengen von 1 bis 7 mol pro Mol organischer Ausgangsstoff verwendet wird, vorzugsweise in Mengen von 1 bis 4 mol pro Mol organischer Ausgangsstoff.

**15.** Verfahren nach einem der Ansprüche 8 bis 14, dadurch gekennzeichnet, daß das Oxidationsmittel in Mengen von 1 bis 10 mol pro Mol Ausgangsstoff verwendet wird, vorzugsweise in Mengen von 1 bis 7 mol pro Mol Ausgangsstoff.

**16.** Verfahren nach einem der Ansprüche 8 bis 15, dadurch gekennzeichnet, daß die Katalysatoren in Mengen von 0,001 bis 0,5 mol pro Mol Ausgangsstoff hinzugegeben werden, vorzugsweise in Mengen von 0,02 bis 0,3 mol pro Mol Ausgangsstoff.

**17.** Verfahren nach einem der Ansprüche 8 bis 16, dadurch gekennzeichnet, daß das Oxidationsmittel langsam und kontinuierlich und regelmäßig zu dem Reaktionsmilieu hinzugegeben wird.

**18.** Verfahren nach einem der Ansprüche 8 bis 17, dadurch gekennzeichnet, daß der nucleophile Ausgangsstoff ausgewählt ist aus:

- aromatischen oder heterocyclischen, (primären, sekundären oder tertiären) aliphatischen organischen Sulfiden und Disulfiden;
- Thiolen;
- Olefinen und gegebenenfalls durch Elektronendonorgruppen aktivierten Acetylenderivaten, wie z.B. Enolen und deren Derivaten (insbesondere Ethern und Enolestern), Enaminen und deren Derivaten, Acetylenethern, Ynaminen...;
- Acrylamiden;

- enolisierbaren Ketonen (β-Diketonen, β-Ketoestern);
- Ketonen oder α,β-ungesättigten Säurederivaten, gegebenenfalls cyclisch bzw. ringförmig, so z.B. Uracil oder Uridin;
- gegebenenfalls kondensierten, mono- oder polycyclischen, homo- oder heteroaromatischen Derivaten, die vorzugsweise mit mindestens einer über Induktion und/oder Mesomerie elektronenspendenden Gruppe substituiert ist, z.B. Halogenbenzolen, Phenolen, Anilinen, Anisolen, Thiophen und Derivaten.

**19.** Verfahren nach einem der Ansprüche 8 bis 18, dadurch gekennzeichnet, daß die Kohlenstoffanzahl des nucleophllen Ausgangsstoffs höchstens 50 beträgt, vorzugsweise 30.

**20.** Verfahren nach einem der Ansprüche 8 bis 19, dadurch gekennzeichnet, daß das Perfluoralkansulfinat vorzugsweise die folgende Formel (I) aufweist

$$R\text{-}(CF_2)_n\text{-}SO_2^- \quad (I)$$

in der:

- R Wasserstoff, eine Alkylgruppe, eine Arylgruppe, ein Chloratom oder ein Fluoratom ist;
- n eine ganze Zahl von 1 bis 8, vorzugsweise von 1 bis 4, ist, vorzugsweise mit der Maßgabe, daß R Fluor oder Chlor darstellt, wenn n gleich 1 ist.